# EUROPEAN PATENT APPLICATION

(11) **EP 1 627 637 A1**
(43) Date of publication of application: **22.02.2006**
(21) Application number: 04734397.5
(22) Date of filing: 21.05.2004
(51) Int. Cl.: A61K 31/496, A61K 31/4725, A61K 47/32, A61K 47/38, A61K 9/08, A61P 31/04, A61P 27/02

(54) **OPHTHALMIC SOLUTION CONTAINING QUINOLONE ANTIMICROBIAL COMPOUND**

(30) Priority: 23.05.2003 JP 2003146621
(71) Applicant: SANTEN PHARMACEUTICAL CO., LTD., Higashiyodogawa-ku, Osaka-shi, Osaka 533-8651 (JP); DAIICHI PHARMACEUTICAL CO., LTD., Chuo-ku, Tokyo 103-8234 (JP)
(72) Inventor: ASADA, Hiroyuki, SANTEN PHARMACEUTICAL CO., LTD., Osaka-shi, Osaka 533-8651 (JP); KIMURA, Akio, SANTEN PHARMACEUTICAL CO., LTD., Osaka-shi, Osaka 533-8651 (JP)
(74) Representative: Grönlund, Tim Linus Wilhelm
(86) International application number: PCT/JP2004/007312
(87) International publication number: WO 2004/103373

(57) **Abstract**

The present invention provides an ophthalmic solution containing as the active ingredient a quinolone antimicrobial compound, wherein at least one member selected from the group consisting of water-soluble vinyl polymeric compounds and water-soluble cellulose derivatives is formulated into the ophthalmic solution to prevent the quinolone antimicrobial compound from precipitating when the ophthalmic solution comes in contact with the lacrimal fluid after instillation.

## Description

### Background of the Invention

The present invention relates to an ophthalmic solution comprising as the active ingredient a quinolone antimicrobial compound or salt thereof, wherein the quinolone antimicrobial compound or salt thereof can be prevented from separating out as a precipitate when coming in contact with the lacrimal fluid by adding at least one member selected from the group consisting of water-soluble vinyl polymeric compounds and water-soluble cellulose derivatives to the formulation for the ophthalmic solution.

The quinolone antimicrobial compounds are considered as excellent antimicrobial agent because of their strong antibacterial action and high safety, so that those compounds are generally used for ophthalmic solutions. Some quinolone antimicrobial compounds are water-soluble, and others, for example, having both an acidic group (e.g., carboxylic acid group or the like) and a basic group (e.g., amino group or the like) as the substituents have a tendency not to be dissolved in water because a betaine structure is formed within the neutral region. Such quinolone antimicrobial compounds include not only ciprofloxacin, but also, for example, sitafloxacin, norfloxacin, lomefloxacin, tosufloxacin, gatifloxacin, amifloxacin, fleroxacin, enoxacin, difloxacin, and temafloxacin (Japanese Patent Nos. 2714597 and 2121924, and Japanese Patent Unexamined Publication (JP Kokai) Sho 53-141286, 57-77683 and the like).

To make the quinolone antimicrobial compounds soluble, various methods are proposed, for example, addition of acid or alkali to adjust the pH value within an acidic region or basic region, use of a solubilizer, and so on. However, when such improved ophthalmic solutions containing the quinolone antimicrobial compound are administered by instillation, the effects obtained by the improvements would be lower than expected by the contact of the ophthalmic solution with the lacrimal fluid having a neutral pH range (pH6 to 8), and consequently the quinolone antimicrobial compound may separate out as a precipitate on the surface of the eyes. It is reported in Am. J. Ophthalmol., 117, pp258-259 (1994) that ciprofloxacin is liberated as a precipitate on the surface of the eyes (corneal epithelium) after instillation of the ciprofloxacin-containing ophthalmic solution.

In the field of ophthalmic solutions, water-soluble vinyl polymeric compounds such as polyvinyl alcohol, polyvinyl pyrrolidone and the like, and water-soluble cellulose derivatives such as hydroxypropyl methyl cellulose and the like are currently used as the thickening agent or dispersing agent.

International Patent Publication WO02/26234 discloses that addition of carboxymethyl cellulose or the like to acyclovir that is found active on the herpes virus can produce an aqueous suspension having suspended particles with uniform particle diameter. Further, there is the description in J. Ocul. Pharmcol. Ther., 17, pp555-563 (2001) that the quinolone antimicrobial compound can be sustainedly released from the quinolone antimicrobial compound-containing ophthalmic solution by blending hydroxypropyl methyl cellulose or the like into the formulation for the ophthalmic solution.

However, there is no description in any of the above-mentioned references suggesting that when a water-soluble vinyl polymeric compound or water-soluble cellulose derivative is blended into the ophthalmic solution containing the quinolone antimicrobial compound, the quinolone antimicrobial compound can be prevented from precipitating even though the ophthalmic solution comes in contact with the lacrimal fluid after instillation.

### Disclosure of Invention

In light of the above-mentioned circumstances, there is a demand for a method for preventing the quinolone antimicrobial compound or the salt thereof from separating out on the surface of the eyes when the quinolone antimicrobial compound-containing ophthalmic solution comes in contact with the lacrimal fluid after instillation.

The inventors of the present invention have intensively studied about the ophthalmic solution containing the quinolone antimicrobial compound. As a result, it has been found that when a water-soluble vinyl polymeric compound or water-soluble cellulose derivative is formulated into the ophthalmic solution containing the quinolone antimicrobial compound, for example, ciprofloxacin, norfloxacin, lomefloxacin, sitafloxacin, tosufloxacin, or the like, precipitation of the quinolone antimicrobial compound can be prevented after the ophthalmic solution is placed in the eyes by instillation and brought into contact with the lacrimal fluid.

Namely, the present invention provides an ophthalmic solution comprising as the active ingredient a quinolone antimicrobial compound or salt thereof, wherein at least one member selected from the group consisting of water-soluble vinyl polymeric compounds and water-soluble cellulose derivatives is further contained in the formulation for the ophthalmic solution to prevent the quinolone antimicrobial compound or salt thereof from separating out as a precipitate when the ophthalmic solution comes in contact with the lacrimal fluid. Also, the present invention provides a method for preventing a precipitate of quinolone antimicrobial compound from separating out after instillation of an ophthalmic solution comprising as the active ingredient the quinolone antimicrobial compound or salt thereof, by blending at least one member selected from the group consisting of water-soluble vinyl polymeric compounds and water-soluble cellulose derivatives into the ophthalmic solution.

### Best Mode for Carrying out the Invention

The quinolone antimicrobial compounds or salts thereof for use in the present invention are not particularly limited so long as they may tend to separate out on the surface of the eyes when coming in contact with the lacrimal fluid. For example, there can be employed ciprofloxacin, sitafloxacin, norfloxacin, lomefloxacin, tosufloxacin, gatifloxacin, amifloxacin, fleroxacin, enoxacin, difloxacin, and temafloxacin. The concentration of the quinolone antimicrobial compound or salt thereof in the ophthalmic solution of the present invention may appropriately be determined based on their medicinal properties with no particular limitation. For example, the concentration may be in the range of 0.001 to 2% (w/v).

The water-soluble vinyl polymeric compounds used in the present invention are not particularly limited so long as they are water-soluble vinyl polymers, and include, for example, polyvinyl alcohol, polyvinyl pyrrolidone (Polyvinyl pyrrolidone K25, Polyvinyl pyrrolidone K30, Polyvinyl pyrrolidone K90 and the like), and carboxyvinyl polymers. Preferably used is polyvinyl alcohol or polyvinyl pyrrolidone.

The water-soluble cellulose derivatives used in the present invention are not particularly limited so long as they are water-soluble cellulose derivatives, and include, for example, hydroxyethyl cellulose, hydroxypropyl methyl cellulose, methyl cellulose, ethyl cellulose, sodium carboxymethyl cellulose, and the like. Preferably used is hydroxypropyl methyl cellulose.

In the present invention at least one selected from the group consisting of water-soluble vinyl polymeric compounds and water-soluble cellulose derivatives may be formulated into the formulation for the ophthalmic solution. It is preferable to obtain the formulation so that at least one member selected from the water-soluble vinyl polymeric compounds and water-soluble cellulose derivatives may be blended at a concentration of 0.01 to 5% (w/v).

The ophthalmic solution of the present invention may further include a tonicity agent, buffering agent, pH adjusting agent, solubilizer, stabilizer, preservative and the like.

Examples of the tonicity agent include glycerin, propylene glycol, sodium chloride, potassium chloride, sorbitol, and mannitol.

Examples of the buffering agent include phosphoric acid, phosphates, citric acid, acetic acid, ε-aminocaproic acid, and trometamol.

Examples of the pH adjusting agent include hydrochloric acid, citric acid, phosphoric acid, acetic acid, sodium hydroxide, potassium hydroxide, boric acid, borax, sodium carbonate, and sodium bicarbonate.

Examples of the solubilizer include Polysorbate 80, polyoxyethylene hydrogenated castor oil 60, and macrogol 400.

Examples of the stabilizer include edetic acid and sodium edetate.

Examples of the preservative (antiseptic) include generally used sorbic acid, potassium sorbate, benzalkonium chloride, benzethonium chloride, methyl parahydroxybenzoate, propyl parahydroxybenzoate, and chlorobutanol. Those preservatives may be used in combination.

Desirably, the ophthalmic solution according to the present invention may be adjusted to pH4.5 to 8.5. The ratio of the osmotic pressure of the ophthalmic solution to that of physiological saline may desirably be set at around 1.0.

The dosage of the ophthalmic solution according to the present invention may appropriately be determined depending upon the symptoms, age, and the like, and the ophthalmic solution may be administered by instillation once to several times a day.

The present invention will now be explained in detail by referring to the examples, which are not intended to be limiting of the present invention.

### Examples

To check the solubility of the ophthalmic solution according to the present invention when coming in contact with the lacrimal fluid, the following ophthalmic solutions were prepared, and the concentration of each quinolone antimicrobial compound was determined after the ophthalmic solution was mixed with the lacrimal fluid artificially prepared.

### Example 1

To 90 mL of physiological saline, 0.1 g of hydroxypropyl methyl cellulose (2910) and 0.3 g of sitafloxacin were successively added. With the addition of a proper amount of diluted hydrochloric acid, the sitafloxacin was dissolved in the mixture. The resultant mixture was adjusted to pH 5.1 by the addition of aqueous sodium hydroxide solution and diluted hydrochloric acid, and the volume of the mixture was then increased to 100 mL by the addition of physiological saline. The mixture was filtered through a membrane filter of 0.22 µm, thereby obtaining an ophthalmic solution.

### Example 2

The same procedure as in Example 1 was repeated except that 0.3 g of hydroxypropyl methyl cellulose (2910) was used, so that an ophthalmic solution was obtained.

### Example 3

The same procedure as in Example 1 was repeated except that the hydroxypropyl methyl cellulose (2910) in an amount of 0.1 g was replaced by polyvinyl pyrrolidone in an amount of 3.0 g, so that an ophthalmic solution was obtained.

### Example 4

The same procedure as in Example 1 was repeated except that the hydroxypropyl methyl cellulose (2910) in an amount of 0.1 g was replaced by polyvinyl alcohol in an amount of 1.0 g, so that an ophthalmic solution was obtained.

### Comparative Example 1

The same procedure as in Example 1 was repeated except that 0.1 g of the hydroxypropyl methyl cellulose (2910) was not used, so that a comparative ophthalmic solution was obtained.

### Example 5

To 90 mL of physiological saline, 0.3 g of hydroxypropyl methyl cellulose (2910) and 0.3 g of ciprofloxacin were successively added. With the addition of a proper amount of diluted hydrochloric acid, the ciprofloxacin was dissolved in the mixture. The resultant mixture was adjusted to pH 4.5 by the addition of aqueous sodium hydroxide solution and diluted hydrochloric acid, and the volume of the mixture was then increased to 100 mL by the addition of physiological saline. The mixture was filtered through a membrane filter of 0.22 µm, thereby obtaining an ophthalmic solution.

### Example 6

The same procedure as in Example 5 was repeated except that 1.0 g of the hydroxypropyl methyl cellulose (2910) was used, so that an ophthalmic solution was obtained.

### Example 7

The same procedure as in Example 5 was repeated except that the hydroxypropyl methyl cellulose (2910) in an amount of 0.3 g was replaced by polyvinyl pyrrolidone in an amount of 3.0 g, so that an ophthalmic solution was obtained.

### Example 8

The same procedure as in Example 5 was repeated except that the hydroxypropyl methyl cellulose (2910) in an amount of 0.3 g was replaced by polyvinyl alcohol in an amount of 1.0 g, so that an ophthalmic solution was obtained.

### Comparative Example 2

The same procedure as in Example 5 was repeated except that 0.3 g of the hydroxypropyl methyl cellulose (2910) was not used, so that a comparative ophthalmic solution was obtained.

### Example 9

To 90 mL of physiological saline, 0.3 g of hydroxypropyl methyl cellulose (2910) and 0.3 g of norfloxacin were successively added. With the addition of a proper amount of diluted hydrochloric acid, the norfloxacin was dissolved in the mixture. The resultant mixture was adjusted to pH 6.4 by the addition of aqueous sodium hydroxide solution and diluted hydrochloric acid, and the volume of the mixture was then increased to 100 mL by the addition of physiological saline. The mixture was filtered through a membrane filter of 0.22 µm, thereby obtaining an ophthalmic solution.

### Example 10

The same procedure as in Example 9 was repeated except that the hydroxypropyl methyl cellulose (2910) in an amount of 0.3 g was replaced by polyvinyl alcohol in an amount of 1.0 g, so that an ophthalmic solution was obtained.

### Comparative Example 3

The same procedure as in Example 10 was repeated except that 0.3 g of the hydroxypropyl methyl cellulose (2910) was not used, so that a comparative ophthalmic solution was obtained.

### Example 11

To 90 mL of a 2.5% concentrated glycerin solution, 1.0 g of hydroxypropyl methyl cellulose (2910) and 1.2 g of lomefloxacin were successively added. With the addition of a proper amount of diluted hydrochloric acid, the lomefloxacin was dissolved in the mixture. The resultant mixture was adjusted to pH 6.3 by the addition of aqueous sodium hydroxide solution and diluted hydrochloric acid, and the volume of the mixture was then increased to 100 mL by the addition of 2.5% concentrated glycerin solution. The mixture was filtered through a membrane filter of 0.22 µm, thereby obtaining an ophthalmic solution.

### Example 12

The same procedure as in Example 11 was repeated except that the hydroxypropyl methyl cellulose (2910) in an amount of 1.0 g was replaced by polyvinyl alcohol in an amount of 1.0 g, so that an ophthalmic solution was obtained.

### Comparative Example 4

The same procedure as in Example 11 was repeated except that 1.0 g of the hydroxypropyl methyl cellulose (2910) was not used, so that a comparative ophthalmic solution was obtained.

### Example 13

To 90 mL of physiological saline, 0.3 g of hydroxypropyl methyl cellulose (2910) and 0.01 g of tosufloxacin were successively added. With the addition of a proper amount of sodium hydroxide aqueous solution, the tosufloxacin was dissolved in the mixture. The resultant mixture was adjusted to pH 5.2 by the addition of diluted hydrochloric acid and sodium hydroxide, and the volume of the mixture was then increased to 100 mL by the addition of physiological saline. The mixture was filtered through a membrane filter of 0.22 µm, thereby obtaining an ophthalmic solution.

### Example 14

The same procedure as in Example 13 was repeated except that the hydroxypropyl methyl cellulose (2910) in an amount of 0.3 g was replaced by polyvinyl alcohol in an amount of 1.0 g, so that an ophthalmic solution was obtained.

### Comparative Example 5

The same procedure as in Example 13 was repeated except that 0.3 g of the hydroxypropyl methyl cellulose (2910) was not used, so that a comparative ophthalmic solution was obtained.

### [Test Method]

15 mL of each of the ophthalmic solutions obtained in Examples 1 to 14 and Comparative Examples 1 to 5 was placed into a 20-mL glass beaker and stirred with a magnetic stirrer. Subsequently, 3 mL of the artificial lacrimal fluid (isotonic phosphoric acid buffer solution: pH 7.5) was added to each ophthalmic solution, followed by stirring. Immediately after addition of the artificial lacrimal fluid, five minutes later and ten minutes later, 2mL of solution was sampled from each ophthalmic solution and the sample was filtered through a membrane filter of 0.22 µm. The concentration of the quinolone antimicrobial compound in each filtrate was determined using high-performance liquid chromatography. The ratio of the concentration of quinolone after a lapse of a given time to the concentration of quinolone obtained immediately after addition of the artificial lacrimal fluid was calculated as a solubility ratio (%). The test results are shown in Tables 1 through 5.

**[Table 1]**

| | | Examples | | | | Comp. Ex. 1 |
|---|---|---|---|---|---|---|
| | | 1 | 2 | 3 | 4 | |
| Sitafloxacin (g) | | 0.3 | 0.3 | 0.3 | 0.3 | 0.3 |
| HPMC*¹ (g) | | 0.1 | 0.3 | | | |
| VP*² (g) | | | | 3.0 | | |
| VA*³ (g) | | | | | 1.0 | |
| pH | | 5.1 | 5.1 | 5.1 | 5.1 | 5.1 |
| Artificial lacrimal fluid | Immediately after addition (%) | 100.0 | 100.0 | 100.0 | 100.0 | 100.0 |
| | 10 min. later (%) | 96.2 | 100.0 | 99.6 | 91.9 | 71.3 |

| | | | | | | |
|---|---|---|---|---|---|---|
| Note | | | | | | |
| *1: Hydroxypropyl methyl cellulose (2910), made by Shin-Etsu chemical Co., Ltd. This also applies to the following. | | | | | | |
| *2: Polyvinyl pyrrolidone, made by BASF Japan Ltd. This also applies to the following. | | | | | | |
| *3: Polyvinyl alcohol, made by Kuraray Co., Ltd. This also applies to the following. | | | | | | |

**[Table 2]**

| | | Examples | | | | Comp. Ex. 2 |
|---|---|---|---|---|---|---|
| | | 5 | 6 | 7 | 8 | |
| Ciprofloxacin (g) | | 0.3 | 0.3 | 0.3 | 0.3 | 0.3 |
| HPMC*¹ (g) | | 0.3 | 1.0 | | | |
| VP*² (g) | | | | 3.0 | | |
| VA*³ (g) | | | | | 1.0 | |
| pH | | 4.5 | 4.5 | 4.5 | 4.5 | 4.5 |
| Artificial lacrimal fluid | Immediately after addition (%) | 100.0 | 100.0 | 100.0 | 100.0 | 100.0 |
| | 10 min. later (%) | 97.9 | 99.0 | 97.4 | 99.1 | 85.8 |

**[Table 3]**

| | | Examples | | Comp. Ex. 3 |
|---|---|---|---|---|
| | | 9 | 10 | |
| Norfloxacin (g) | | 0.3 | 0.3 | 0.3 |
| HPMC*¹ (g) | | 0.3 | | |
| VA*³ (g) | | | 1.0 | |
| pH | | 6.4 | 6.4 | 6.4 |
| Artificial lacrimal fluid | Immediately after addition (%) | 100.0 | 100.0 | 100.0 |
| | 10 min. later (%) | 99.7 | 96.8 | 72.4 |

**[Table 4]**

| | | Examples | | Comp. Ex. 4 |
|---|---|---|---|---|
| | | 11 | 12 | |
| Lomefloxacin (g) | | 1.2 | 1.2 | 1.2 |
| HPMC*¹ (g) | | 1.0 | | |
| VA*³ (g) | | | 1.0 | |
| pH | | 6.3 | 6.3 | 6.3 |
| Artificial lacrimal fluid | Immediately after addition (%) | 100.0 | 100.0 | 100.0 |
| | 10 min. later (%) | 99.0 | 99.8 | 96.4 |

**[Table 5]**

| Examples | | 13 | 14 | Comp. Ex. 5 |
|---|---|---|---|---|
| Tosufloxacin (g) | | 0.01 | 0.01 | 0.01 |
| HPMC*¹ (g) | | 0.3 | | |
| VA*³ (g) | | | 1.0 | |
| pH | | 5.2 | 5.2 | 5.2 |
| Artificial lacrimal fluid | Immediately after addition (%) | 100.0 | 100.0 | 100.0 |
| | 10 min. later (%) | 97.2 | 89.3 | 16.4 |

As is apparent from the test results shown in Tables 1 to 5, the ophthalmic solutions where a water-soluble vinyl polymeric compound or water-soluble cellulose derivative, such as polyvinyl alcohol or hydroxypropyl methyl cellulose is added to the quinolone antimicrobial compounds such as sitafloxacin, ciprofloxacin, norfloxacin and the like can effectively prevent the quinolone antimicrobial compounds from precipitating when mixed and stirred with the artificial lacrimal fluid.

As can be seen from the results of Examples 1 to 14 and Comparative Examples 1 to 5, when at least one member selected from the group consisting of water-soluble vinyl polymeric compounds and water-soluble cellulose derivatives is formulated into the ophthalmic solution containing as the active ingredient the quinolone antimicrobial compound or salt thereof, it is possible to prevent the precipitation of the quinolone antimicrobial compound or salt thereof even though the ophthalmic solution comes in contact with the lacrimal fluid after instillation.

## Claims

1. An ophthalmic solution comprising as an active ingredient a quinolone antimicrobial compound or a salt thereof, wherein at least one member selected from the group consisting of water-soluble vinyl polymeric compounds and water-soluble cellulose derivatives is formulated into the ophthalmic solution to prevent the quinolone antimicrobial compound or the salt thereof from precipitating when coming in contact with lacrimal fluid.

2. The ophthalmic solution as claimed in Claim 1, wherein the quinolone antimicrobial compound is ciprofloxacin, norfloxacin, lomefloxacin, sitafloxacin, gatifloxacin or tosufloxacin.

3. The ophthalmic solution as claimed in Claim 1, wherein the water-soluble vinyl polymeric compound is polyvinyl alcohol or polyvinyl pyrrolidone.

4. The ophthalmic solution as claimed in Claim 1, wherein the water-soluble cellulose derivative is hydroxypropyl methyl cellulose.

5. The ophthalmic solution as claimed in Claim 1, wherein the quinolone antimicrobial compound or the salt thereof is contained at a concentration of 0.001 to 2% (w/v), and at least one member selected from the group consisting of the water-soluble vinyl polymeric compounds and the water-soluble cellulose derivatives is contained at a concentration of 0.01 to 5% (w/v).

6. A method for preventing a precipitate of quinolone antimicrobial compound from separating out after instillation of an ophthalmic solution comprising as an active ingredient the quinolone antimicrobial compound or salt thereof, comprising adding at least one member selected from the group consisting of water-soluble vinyl polymeric compounds and water-soluble cellulose derivatives into the ophthalmic solution.
